# EUROPEAN PATENT SPECIFICATION

(11) **EP 3 516 558 B1**
(45) Date of publication and mention of the grant of the patent: **26.02.2020**
(21) Application number: 17768441.2
(22) Date of filing: 15.09.2017
(51) Int. Cl.: G16H 50/50

(54) **SYSTEM AND METHOD FOR ASSESSING OUTFLOW TRACT OBSTRUCTION OF A HEART OF A SUBJECT**
SYSTEM UND VERFAHREN ZUR BEWERTUNG VON AUSFLUSSTRAKTOBSTRUKTION EINES HERZENS EINES PATIENTEN
SYSTÈME ET PROCÉDÉ D'ÉVALUATION DE L'OBSTRUCTION DES VOIES D'ÉVACUATION DU C UR D'UN SUJET

(30) Priority: 23.09.2016 EP 16190375
(43) Date of publication of application: 31.07.2019
(73) Proprietor: Koninklijke Philips N.V., 5656 AE Eindhoven (NL)
(72) Inventor: WEKEL, Tilman, 5656 AE Eindhoven (NL); STEHLE, Thomas Heiko, 5656 AE Eindhoven (NL); WEESE, Rolf Jürgen, 5656 AE Eindhoven (NL)
(74) Representative: Versteeg, Dennis John
(86) International application number: PCT/EP2017/073216
(87) International publication number: WO 2018/054762

(56) References cited:
- US-A1- 2010 240 996
- US-A1- 2015 112 901
- US-A1- 2016 166 332
- US-A1- 2016 228 190

## Description

### FIELD OF THE INVENTION

The present invention relates to a system for assessing outflow tract obstruction of a heart of a subject, a corresponding method and a corresponding computer program. It finds particular application in assessing the potential obstruction of the outflow tract caused by mitral valve replacement procedures, in particular caused by a mitral valve implant. However, it is to be understood that the present invention also finds applications in other fields and is not necessarily limited to the above mentioned application.

### BACKGROUND OF THE INVENTION

Mitral valve regurgitation is among the most common heart diseases of elderly patients, wherein mitral valve replacement is one of the gold standards for a treatment of mitral valve regurgitation patients. However, one of the typical complications after mitral valve replacement is obstruction of the left ventricular outflow tract (LVOT). The mitral valve implant dilates or moves the mitral valve leaflet towards the aortic valve and thereby elongates and narrows the LVOT, which can drastically affect the blood flow and thus the pressure drop between left ventricle and aorta.

It is therefore desired to assess such as to measure or even predict the obstruction caused by a mitral valve implant. Up to now a clinician identifies a suitable slice of a computed tomography (CT) volume and marks the outflow tract before and/or after the intervention. This procedure is error prone, since it highly depends on the selection of the slice, and no well-defined metric or criterion exists for a quantitative evaluation.

US 2016/0166332 A1 discloses a method for evaluating the placement of a prosthetic heart valve in a structure of interest. The method comprises acquiring one or more depictions of an anatomical region of interest that includes the structure of interest, wherein each depiction shows the structure of interest and/or the blood pool volume of the left ventricular outflow tract (LVOT) of the patient's heart. The method further comprises designating one or more positions in at least one of the one or more depictions wherein each position corresponds to a respective position in the structure of interest at which the prosthetic valve may be placed. The method still further comprises predicting, for each of the one or more designated positions, an amount of blood flow obstruction through the LVOT of the patient's heart that would occur if the prosthetic valve was to be placed at a corresponding position in the structure of interest.

### SUMMARY OF THE INVENTION

It is an object of the present invention to provide a system and a method, which allow for an improved assessing of an outflow tract obstruction of a heart of a subject.

In a first aspect of the present invention a system for assessing outflow tract obstruction of a heart of a subject is provided, wherein the system comprises: a geometrical model providing unit for providing a geometrical model of the heart; a volumetric image providing unit for providing a volumetric image of the heart; a first geometrical model adaptation unit for adapting the geometrical model to the volumetric image to obtain an adapted geometrical model; a second geometrical model adaptation unit for providing an implant model of an implant and for constructing the implant model into the adapted geometrical model to obtain an enhanced adapted geometrical model; a trajectory curve determination unit for determining a trajectory curve through an outflow tract from at least one of the adapted geometrical model and the enhanced adapted geometrical model, and an outflow tract obstruction assessing unit for assessing outflow tract obstruction based on the adapted geometrical model, the enhanced adapted geometrical model and the trajectory curve. The trajectory curve approximates a center line through the outflow tract and comprises an interpolation, such as an interpolating spline curve, between anatomical landmarks, wherein the anatomical landmarks include the center of the aortic valve plane, a center of the extruded mitral valve plane and the apex, wherein the anatomical landmarks are comprised in the geometrical model and/or determined based on the geometrical model.

Since the outflow tract obstruction assessing unit assesses outflow tract obstruction based on two models, wherein one model is an adapted geometrically model and the further model is an enhanced adapted geometrical model, an assessment of outflow tract obstruction can be facilitated through, for instance, a comparison of both models. Instead of selecting a two dimensional slice of the volumetric image of the heart, a geometrical model of the heart is adapted to the volumetric image, i.e. based on information of all dimensions of the volumetric image.

Preferentially, all details of the volumetric image can advantageously be employed for adapting the geometrical model. Since the adapted geometrical model is further enhanced by constructing an implant model of an implant into the adapted geometrical model, a second model is obtained, which differs from the adapted geometrical model in that it further comprises the implant model, which is constructed therein. The adapted geometrical model and the enhanced adapted geometrical model thereby provide a common base for assessment, i.e. the adapted geometrical model, which provides for a facilitated assessment of outflow tract obstruction.

Preferentially, the trajectory curve is a geometrical function describing a curve through the outflow tract. The trajectory curve is constructed based on the geometrical model of the heart, which has been adapted to the volumetric image, and additional anatomical landmarks which can either be encoded into the geometrical model or, alternatively, or additionally, be computed based on the geometrical model. Since the outflow tract obstruction assessing unit assesses the outflow tract obstruction based on the trajectory curve, outflow tract obstruction can be related with a path through the outflow tract, wherein the path coincides with the trajectory curve.

In summary, there is an improved assessing of obstruction of the outflow tract so achieved based on the combination of the adapted geometrical model, the enhanced adapted geometrical model and the trajectory curve through the outflow tract.

The subject is preferentially a human patient. However, it can also be an animal.

Preferentially a geometrical model comprises an explicit definition of the shape of the heart. It is known that geometric models of, for instance, organs can be adapted to image data and used for, for instance, segmenting objects.

Preferentially, the volumetric image of the heart is an image of the heart obtained with any suitable imaging modality, which allows for a three dimensional volumetric view of the heart. Preferentially, without being limited, the volumetric image is obtained with at least of one magnetic resonance imaging (MRI), computed tomography (CT) or nuclear medicine, such as single photon emission computed tomography (SPECT).

In an embodiment the outflow tract is the left ventricular outflow tract (LVOT) of the heart of the subject. However, in other embodiments the outflow tract can also be a different tubular structure, for instance a different outflow tract, such as the right ventricular outflow tract (RVOT).

In an embodiment the implant model comprises a model of a mitral valve implant. The mitral valve implant is a common implant in the field of surgeries related to the heart. However, in other embodiments, the implant model can also comprise a model of a different implant, such as of an aortic valve implant.

In an embodiment at least one of the geometrical model and the implant model is a 3D model.

Preferentially, both the geometrical model and the implant model are provided as a 3D model. In other embodiments, at least one of the geometrical model and the implant model, preferentially the geometrical model, is provided as a four dimensional model, i.e. provided for at least two different positions in time corresponding to different phases of the beating heart of the subject. Since the outflow tract of the heart can change over the heart beat cycle of the heart, by employing a geometrical model which considers more than one single point during the cycle, the point which has most influence to outflow tract obstruction can be considered. In other embodiments, a plurality of 3D models is provided for different phases which are than considered individually.

The second geometrical model adaptation unit can in one embodiment be adapted to determine an annulus ring of a valve in the adapted geometrical model, wherein the annulus ring defines a plane comprising a center position and a normal vector, and to position the implant model with respect to the annulus ring.

The annulus ring preferentially corresponds to the part of the geometrical model, where the implant, such as the mitral valve implant, will be implanted to. In other words, the annulus ring preferentially corresponds to the anatomical structure at which the mitral valve leaves originate. The annulus ring can be predefined in the geometrical model, i.e. the geometrical model can already be provided with additional information indicating the anatomical structures contained in the geometrical model. In other embodiments, the annulus ring can also be determined based on the geometry of the geometrical model.

Since the annulus ring defines the opening, for instance, through the mitral valve, a center position in the plane, in which the annulus ring lies, can be defined together with a normal vector. The implant model can then be positioned with respect to the annulus ring.

The second geometrical model adaptation unit can further in one embodiment be adapted to allow a translation and/or rotation of the implant model based on an input of a user.

An input of a user can preferentially be provided by means of any known user input unit. By allowing a translation and/or rotation of the implant model after positioning the implant model with respect to the annulus ring, the positioning of the implant model within the adapted geometrical model can further be improved.

The second geometrical model adaptation unit can in one embodiment be adapted to provide the implant model as a mesh template, wherein the mesh template is parameterized based on at least one of a size, width and length, and/or to construct a geometry based on an annulus ring geometry or a mitral valve of the adapted geometrical model.

In case the implant model is provided as a mesh template, the mesh template can advantageously be adapted to the annulus ring determined in the adapted geometrical model. To this end, at least one of a size, width and length of the mesh template, which are included as parameters, can be employed. In different embodiments, the implant model is constructed based on an annulus ring geometry or the mitral valve, i.e. created using geometrical considerations. However, in other embodiments, also a combination of both approaches or a different approach for providing the implant model is contemplated.

The outflow tract obstruction assessing unit can in one embodiment be adapted to provide a relative quantity indicating the obstruction before and after mitral valve replacement based on the difference between the enhanced adapted geometrical model and the adapted geometrical model and based on the trajectory curve.

In general, after mitral valve replacement, parts of the mitral valve implant occupy the outflow tract. Since the outflow tract obstruction assessing unit can provide relative quantity indicating this relative obstruction, a quantitative indication for the obstruction due to mitral valve replacement can be obtained.

The interrelation between adapted geometrical model, enhanced adapted geometrical model and the outflow tract is utilized based on the trajectory curve. Since the enhanced adapted geometrical model is determined based on the adapted geometrical model and preferentially shares the same topology and/or additional information, such as landmark information and/or labels, these models can be directly related and the assessment of the outflow tract is facilitated.

The trajectory curve determination unit can in one embodiment be configured to determine the trajectory curve based on the geometrical model of the heart from a central point of the aortic valve plane to the apex.

Preferentially, the central point of the aortic valve plane is obtained the same way in the geometrical model as the annulus ring. More specifically, the central point of the aortic valve plane can either be encoded into the geometrical model or be computed based on the geometrical model. Preferentially, the geometrical model providing unit and/or the first geometrical model adaptation unit is adapted to extract anatomical planes such as for the valves as well as important landmarks such as the apex from the geometrical model.

The trajectory curve can in one embodiment approximate a center line through the outflow tract and comprises an interpolation, such as an interpolating spline curve, between anatomical landmarks, wherein the anatomical landmarks include a center of the aortic valve plane, a center of the extruded mitral valve plane and the apex, wherein the anatomical landmarks are comprised in the geometrical model or determined based on the geometrical model.

In this embodiment, the anatomical landmarks include the center of the aortic valve plane, the center of the extruded mitral valve plane and the apex. However, in other embodiments, also further or alternative landmarks can be considered. In general, the center line through the outflow tract is not unambiguously derivable. For this reason, the trajectory curve preferentially approximates the center line through the outflow tract by means of an interpolation. However, in other embodiments, also other definitions and methods of constructing a center line through the outflow tract are contemplated.

In an embodiment the trajectory curve determination unit is adapted to determine the trajectory employing at least one of a modified distance transform, a medial axis transform, or a voronoi decomposition, in particular in case the geometrical model is provided in the form of a mesh model comprising triangles vertices.

In this embodiment, the geometrical model of the heart does not have to be semantically enriched, i.e. does not have to comprise anatomical landmarks and the like encoded therein. For instance, the geometrical model of the heart can in this embodiment be provided as a mesh consisting of triangles and vertices, wherein the center line is constructed based on this mesh. The exemplary algorithms mentioned above infer a skeleton from three dimensional, discrete structures. However, in other embodiments, also other inferring algorithms can be used for constructing the trajectory curve, in particular the center line through the outflow tract.

In an embodiment the system further comprises a slice plane determination unit for determining a slice plane corresponding to a point of the trajectory curve, wherein the normal of the slice plane coincides with a tangent to the trajectory curve in the point.

Preferentially, the plane is centered at the trajectory curve. Since the trajectory curve provides a geometrical function which is guiding through the outflow tract and the slice planes correspond to planes perpendicular to a point on the trajectory curve, the slice planes comprise cross sectional views of the outflow tract along the trajectory through the outflow tract.

In an embodiment the system further comprises a cross section determination unit for determining a cross section of the outflow tract based on the slice plane.

Determining the cross section preferentially includes delineating the left ventricular structure from surrounding elements of the module. Preferentially, the geometrical model already includes the ventricular wall as an element encoded therein, such that determining the cross section of the outflow tract based on the slice plane is facilitated.

The cross section determination unit can in one embodiment be configured to determine the cross section of the outflow tract based on at least one of a flood fill or a region growing.

While flat fill or region growing are explicitly listed as algorithms for determining an area of arbitrary shape, such as the cross section of the outflow tract comprised in the slice plane, also other algorithms for determining such arbitrarily shaped surfaces, which are well known to the person skilled in the art, can be employed.

For instance, in other embodiments such as in a situation, in which the cross section to be filled is likely to be given by a piecewise disconnected boundary curve, i.e. the cross section can be comprised of a plurality of parts which are not connected, algorithms which directly operate on a set of points, such as alpha shapes can be employed. The use of alpha shapes has been described, for instance, in N. Akkiraju et al. "Alpha Shapes: Definition and Software", in Proceedings of the International Computational Geometry Software Workshop 1995, Minneapolis.

Preferentially, the cross section is determined for both the adapted geometrical model and the enhanced adapted geometrical model, i.e. for both the model comprising the implant model and not comprising the implant model.

In an embodiment the system allows the user to carry out adaptations to the underlying heart model and/or the implant model. Based on these adaptations, the cross section determination unit can then be adapted to reevaluate the cross sections of the outflow tract.

In an embodiment the system further comprises an elevation profile determination unit for determining an elevation profile of a parameter relating to the slice plane for different points of the trajectory curve, and a display unit, wherein the display unit is configured to simultaneously display the slice plane and the elevation profile and to indicate the point, to which the slice plane corresponds, on the elevation profile.

Since the display unit is configured to display the slice plane together with the evaluation profile, a user, such as a physician, can at the same time evaluate the slice plane and relevant parameters relating to this slice plane. Preferentially, the physician can adjust the relevant parameters to be displayed in the display unit. Furthermore, the physician can operate an input unit and move along the trajectory curve, wherein a slice plane corresponding to the current position along the trajectory curve is at the same time displayed and indicated in the evaluation profile.

The cross section determination unit preferentially determines cross sections for a predefined set of equally distanced positions along the trajectory curve.

The parameter can in one embodiment be at least one of an area, a perimeter or a compactness measure of the outflow tract in the slice plane, such as shape factors.

Shape factors include, without being limited, a circularity or an elongation shape factor. In other embodiments, alternative or additional parameters which are used for the elevation profile can be used.

The elevation profile can in one embodiment be based on the parameter for at least one of the adapted geometrical model and the enhanced adapted geometrical model.

The elevation profile can either indicate the parameter for the adapted geometrical model, for the enhanced adapted geometrical model or for a difference between enhanced adapted geometrical model versus adapted geometrical model. In other words, and in case the parameter is exemplarily taken to be an area, the elevation profile can indicate a difference in area of the outflow tract between the enhanced adapted geometrical model comprising the implant and the adapted geometrical model without the implant such that an effect of outflow tract obstruction based on the implant model can directly be extracted from the elevation profile.

In an embodiment the system further comprises a weighting function determination unit for determining a weighting function for at least one slice plane based on at least one of the geometrical model, the adapted geometrical model and the enhanced adapted geometrical model, wherein the weighting function accounts for non-linear fluid mechanics of the outflow tract, and wherein the outflow tract obstruction assessing unit is adapted to assess the outflow tract obstruction based on the weighting function.

Since the outflow tract obstruction is assessed based on the weighting function, non-linear fluid mechanics can be accounted for and an assessment of the outflow tract obstruction is more accurately possible then by considering the volumetric obstruction based on the implant model alone. In other words, instead of determining the obstruction directly, the outflow tract obstruction is evaluated based on the weighing function, which can preferentially be indicative of a pressure drop over the outflow tract.

The volume of the outflow tract can in one embodiment be represented as a set of cross sections determined based on slice planes along the trajectory curve, wherein the outflow tract obstruction assessing unit is adapted to determine an outflow tract geometry based on a volume integral of the weighting function over the outflow tract.

Preferentially, the outflow tract can be represented as a sum of cross sections along the trajectory curve. The integration volume, over which the weighting function is to be integrated, is thereby the entire outflow tract. However, in other example, also parts of the outflow tract can be employed for integrating the weighting function.

The weighting function determination unit can in one embodiment be adapted to determining the weighting function based on a sum of a first contribution, wherein the first contribution is a longitudinal distance along the trajectory curve, and a second contribution, wherein the second contribution is determined in dependence of a distance along the slice plane from the trajectory curve.

This preferred definition of the weighting function is based on the finding, based on fluid mechanics, that obstructions affect the blood flow more if they are closer to the aortic valve and, likewise, closer to the center line of the outflow tract, i.e. closer to the trajectory curve. Preferentially, generally non-linear functions, which represent weights of both the first and second contribution, which in sum result in the overall weighting function, are to be determined in the course of a calibration step. Further preferentially, the two calibration functions can be approximated, such as by polynomial functions. Thereby, a wide range of possible relationships between distances and effects on the pressure drop are covered, wherein parameters of the polynomial calibration functions can be learned by, for instance, cross validation over a preselected set of functions and parameter ranges. Using polynomial functions as approximations for the contributions of the weighting function allows a facilitated computation and calibration. However, in other embodiments also different approximation functions can be used.

The weighting function determination unit can in one embodiment be adapted to determine a parameterized weighting function and to calibrate the parameterized weighting function based on flow simulations such that as a calibration result the parameterized weighting function approximates the result of the flow simulation.

Preferentially, a flow simulation allows to simulate and predict physical quantities of the heart. However, applying flow simulations directly in the system for assessing outflow tract obstructions, such as during online use in a clinical environment, is not possible, since it is a time consuming and complicated task to complete which is unfeasible for applications in clinical environments. Yet, using a parameterized weighting function, which approximates the result of the flow simulation, the drawbacks of setting up and carrying out the flow simulation in a clinical environment is avoided. Nevertheless, the accuracy obtainable with flow simulations can be ported to the system for assessing out flow tract obstructions also for the online use during a clinical session. For instance, a set of training examples including geometrical models with and without implemented implant models is used as an input, wherein the simulation is set up to predict the pressure drop between aortic valve and left ventricle. The result is taken as a ground truth and used to determine the parameter of the weighting function based on, for instance, regression and cross validation.

The geometrical model providing unit, the volumetric image providing unit, the first geometrical model adaptation unit, the second geometrical model adaptation unit, the outflow tract obstruction assessing unit, the trajectory curve determination unit, the slice plane determination unit, the cross section determination unit, the elevation profile determination unit and the weighting function determination unit can in one embodiment be provided in one or more processors that are arranged in the same or different physical devices. More precisely, the first geometrical model adaptation unit, the second geometrical model adaptation unit, the outflow tract obstruction assessing unit, the trajectory curve determination unit, the slice plane determination unit, the cross section determination unit, the elevation profile determination unit and the weighting function determination can in one embodiment be provided together with the geometrical model providing unit and/or the volumetric image providing unit and/or the display unit and/or the input unit in a single device or in a different embodiment be distributed over multiple devices.

In one embodiment, one, more or all of geometrical model providing unit, the volumetric image providing unit, the first geometrical model adaptation unit, the second geometrical model adaptation unit, the outflow tract obstruction assessing unit, the trajectory curve determination unit, the slice plane determination unit, the cross section determination unit, the elevation profile determination unit and the weighting function determination are provided at a server, which is adapted to communicate with the rest of the system for assessing outflow tract obstruction by suitable communication means, for instance via the Internet.

In a further aspect a method for assessing outflow tract obstruction of a heart of a subject is provided, the method comprising a) providing a geometrical model of the heart, b) providing a volumetric image of the heart, c) adapting the geometrical model to the volumetric image to obtain an adapted geometrical model, d) providing an implant model of an implant and for constructing the implant model into the adapted geometrical model to obtain an enhanced adapted geometrical model, e) determining a trajectory curve through an outflow tract from at least one of the adapted geometrical model and the enhanced adapted geometrical model, and f) assessing outflow tract obstruction based on the adapted geometrical model, the enhanced adapted geometrical model and the trajectory curve. In a further aspect a computer program for assessing outflow tract obstruction of a heart of a subject is provided, the computer program comprising program code means for causing a system as defined in claim 1 to carry out the method as defined in claim 23, when the computer program is run on the system.

It shall be understood that the system for assessing of claim 1, the method for assessing of claim 14, and the computer program of claim 15, have similar and/or identical preferred embodiments, in particular, as defined in the dependent claims.

It shall be understood that a preferred embodiment of the present invention can also be any combination of the dependent claims or above embodiments with the respective independent claim.

These and other aspects of the invention will be apparent from and elucidated with reference to the embodiments described hereinafter.

### BRIEF DESCRIPTION OF THE DRAWINGS

In the following drawings:
Fig. 1 schematically and exemplarily shows an embodiment of a system for assessing outflow tract obstruction of a heart of a subject,
Fig. 2A and 2B schematically and exemplarily show a perspective view on an enhanced adapted geometrical model,
Fig. 3A and 3B schematically and exemplarily illustrate the outflow tract obstruction due to mitral valve replacement,
Fig. 4 schematically and exemplarily illustrates the operation of a trajectory curve determination unit,
Fig. 5 schematically and exemplarily illustrates the operation of a slice plane determination unit,
Fig. 6 exemplarily illustrates the operation of a cross section determination unit,
Fig. 7 schematically and exemplarily illustrates the operation of an elevation profile determination unit,
Fig. 8 schematically and exemplarily illustrates a perspective view on an enhanced adapted geometric model,
Fig. 9 schematically and exemplarily illustrates the definition of contributions to a weighting function in two planes, and
Fig. 10 schematically and exemplarily illustrates a flowchart of a method for assessing outflow tract obstruction of a heart of a subject according to the invention.

### DETAILED DESCRIPTION OF EMBODIMENTS

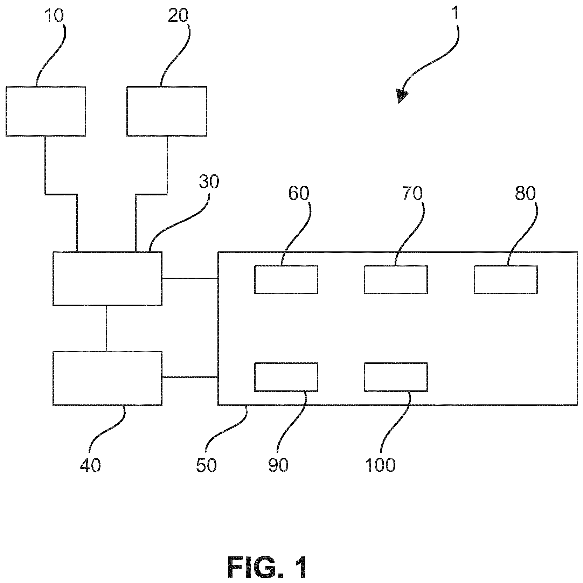

Fig. 1 schematically and exemplarily shows an embodiment of a system 1 for assessing outflow tract obstruction of a heart of a subject. System 1 comprises a geometrical model providing unit 10, a volumetric image proving unit 20, a first geometrical model adaptation unit 30, a second geometrical model adaptation 40, an outflow tract obstruction assessing unit 50, a trajectory curve determination unit 60, a slice plane determination unit 70, a cross section determination unit 80, an elevation profile determination unit 90, and a weighting function determination unit 100. All of the units of system 1 can be provided at a single or different and/or remote locations, such as implemented on a single computer or on a plurality of computers distributed over a network, such as the Internet.

Geometrical model providing unit 10 can in this example provide a geometrical model of the heart of a human or animal subject. The geometrical model can, for instance, be an enriched model, which includes anatomical landmarks and structures included within the model.

Volumetric image providing unit 20 is in this example adapted to provide a volumetric image of the heart, which is provided on a storage within volumetric image providing unit 20 or a different location. In other examples, volumetric image providing unit 20 can also include a volumetric image acquiring unit, wherein volumetric image providing unit 20 is then adapted to provide the volumetric image of the heart acquired by the volumetric image acquiring unit. In other words, volumetric image providing unit 20 can provide a previously acquired volumetric image or can obtain a volumetric image by itself. All suitable medical imaging techniques as known in the art for obtaining a volumetric image of the heart are contemplated and can advantageously be employed in volumetric image providing unit 20.

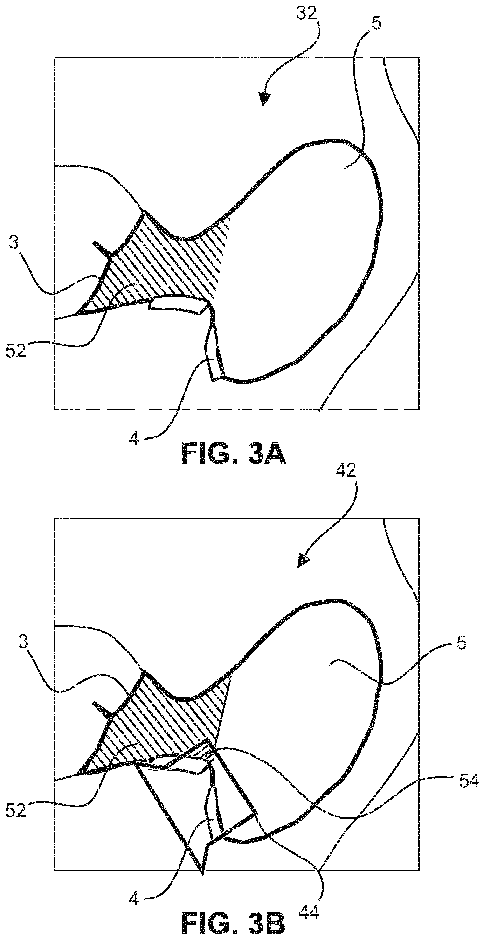

Geometrical model providing unit 10 provides a geometrical model to first geometrical model adaptation unit 30. First geometrical model adaption unit 30 is further configured to be provided with a volumetric image from volumetric image providing unit 20. Based on the geometrical model and the volumetric image, first geometrical model adaption unit 30 adapts the geometrical model to the volumetric image to obtain an adapted geometrical model 30 (cf. Fig. 3, for instance).

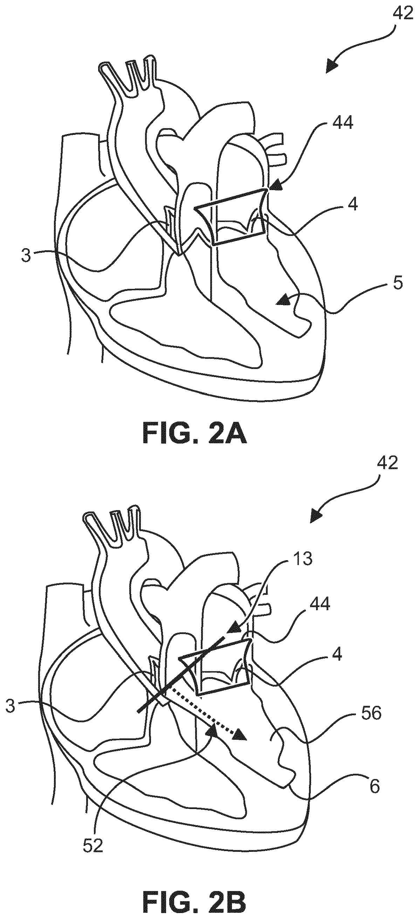

Based on adapted geometrical model 32, second geometrical model adaption unit 40 provides an implant model 44 (cf. Fig. 2, for instance) and constructs implant model 44 into adapted geometrical model 32 to obtain an enhanced adapted geometrical model 42.

Outflow tract obstruction assessing unit 50 assesses outflow tract obstruction based on adapted geometrical model 32, received from first geometrical model adaption unit 30, and enhanced adapted geometrical model 42, received from second geometrical model adaption unit 40. While in this example trajectory curve determination unit 60, slice plane determination 70, cross section determination unit 80, elevation profile determination unit 90 and weighting function determination unit 100 are illustrated as being arranged within outflow tract obstruction assessing unit 50, in other embodiments one, more or all of the units illustrated as lying within outflow tract obstruction assessing unit 50 can be provided independently.

There is no restriction as to communication among the units contained in system 1 and additional communication paths and links, which are not explicitly depicted in Fig. 1, can be introduced as desired by the skilled person.

Second geometrical model adaptation unit 40 is adapted to position implant model 44 with respect to the annulus ring of adapted heart model 32. The annulus ring defines the plane at which the mitral valve originates, and is given by a center location and a normal vector. This center location and normal vector is used for positioning implant model 44 within adapted geometrical model 32. System 1 can provide methods or tools to move and/or rotate implant model 44 arbitrarily in three dimensions or aligned to the annulus geometry after it has been positioned by second geometrical model adaption unit 40. Outflow tract obstruction assessing unit 50 is then adapted to calculate the outflow tract obstruction in an online manner, i.e. substantially in real-time, as a relative quantity which relates obstruction of the outflow tract before and after the - virtual - valve replacement.

The assessment of outflow tract obstruction assessing unit 50 and the further units of system 1 will be described in more detail with reference to exemplary embodiments as illustrated in Figs. 2 - 9.

Fig. 2a and 2b each illustrate schematically and exemplarily a perspective view on enhanced adapted geometrical model 42 in which an aortic valve 3, a mitral valve 4, a left ventricle 5 and the apex 6 of the heart is labeled. Overlaid onto mitral valve 4 is shown the implant model 44 in the example.

Implant model 44 has been positioned in place based on the plane defined by the annulus structure corresponding to mitral valve 4. Implant model 44 has been aligned with the center and the normal of this plane.

Fig. 2b further illustrates the direction of the outflow tract 52 and the outflow tract volume 56. Outflow tract 52 is based on an aortic valve plane 13, which has been placed in the plane of aortic valve 3. Outflow tract volume 56 can be, for instance, calculated as a weighted volume integral over outflow tract 52 from aortic valve plane 13 to the apex 6.

Figs. 3a and 3b schematically and exemplarily illustrate the difference between adapted geometrical model 32 and enhanced adapted geometrical model 42 in a two dimensional slice view through the left ventricle 5, respectively. In addition to adapted geometrical model 32 depicted in Fig. 3a, enhanced adapted geometrical model 42 shown in Fig. 3b further comprises implant model 44 aligned with mitral valve 4. Outflow tract 52 is highlighted in both images. In the case of Fig. 3b it can be seen that a part of outflow tract 52 is obstructed, indicated as outflow tract obstruction 54. The difference between adapted geometrical model 32 and enhanced adapted geometrical model 42 can thus be used for assessing outflow tract obstruction.

Next, one example of generating and placing implant model 44 into adaoted geometrical model 32 is described in more detail. In this example, implant model 44 is a model of a mitral valve implant and is defined as the extruded d-shaped annulus of mitral valve 4, i.e. with a rather simple geometric form with a d-shaped cross section. As such, implant model 44 can be regarded a generic place holder for many different kinds of mitral valve implants. The generic implant model 44 can then quickly be parametrized by size, length, tilt and relative position to the mitral valve 4. Thereby, it is not necessary to obtain a specific model of the final implant, which can be very hard to obtain from the manufacturers. Generic implant model 44 can advantageously be used to plan mitral valve replacement procedures and their effect on the LVOT.

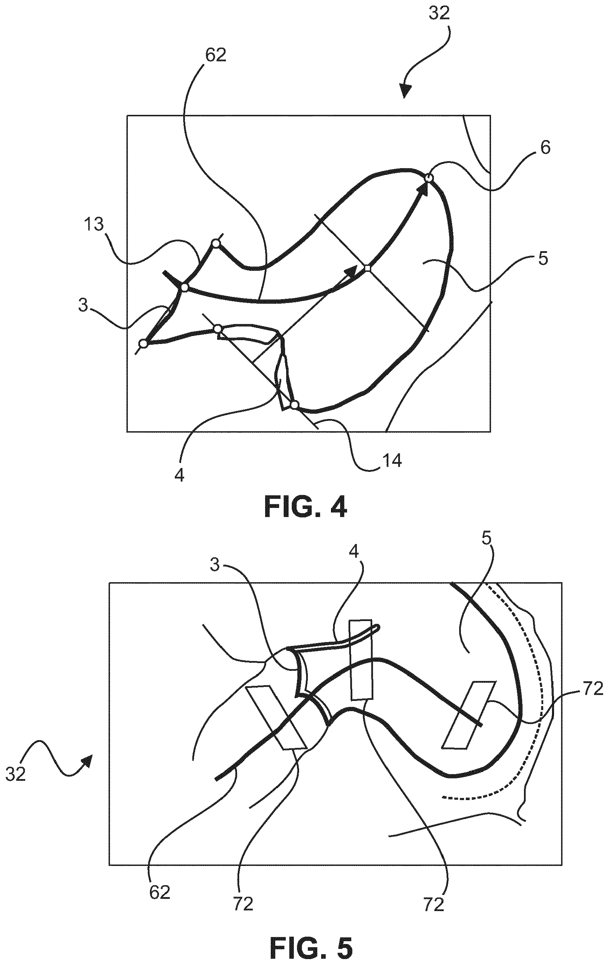

Fig. 4 schematically and exemplarily illustrates the operation of trajectory curve determination unit 60 in the example of adapted geometrical model 32. Trajectory curve determination unit 60 determines a trajectory curve 62 from the center of aortic valve plane 13 to apex 6, wherein trajectory curve 62 approximates the center line of the outflow tract throughout left ventricle 5.

Trajectory curve 62 is constructed based on the geometrical model of the heart itself and additional anatomical landmarks that can either be encoded into the model or computed based on the model. Trajectory curve 62 is in this example defined by an interpolating spline curve, wherein the control points are given, as briefly indicated, by the center of aortic valve plane 13, the center of the extruded mitral valve plane 14 and the apex 6. Once the geometrical model has been adapted to the volumetric image and adapted geometrical model 32 has been obtained, supporting structures can be computed in a straight forward manner. In case the geometrical model is not semantically enriched but only given as a mesh, the centerline could also be constructed by applying algorithms, which infer a skeleton from three dimensional, discrete structures.

It should be noted that mitral valve plane 14 is in this example a virtual plane that is preferentially automatically placed into the model. It is used to define measurements, such as the projected width or length of the mitral valve annulus. Although placed automatically, the mitral valve plane 14 preferentially can be changed afterwards by the user.

Trajectory curve 62 approximates, as already discussed above, the center line of the outflow tract. Preferably, the control points of trajectory curve 62 can be modified and/or improved by the user, such as exemplified for mitral valve plane 14 above, in order to manually optimize trajectory curve 62. By repeated interpolation of the control points, the control points can be modified by the user, i.e. the user can change the shape of the curve, while maintaining its smoothness.

Fig. 5 schematically and exemplarily illustrates the operation of slice plane determination unit 70, which is adapted to construct slice planes perpendicular to trajectory curve 62. Fig. 5 illustrates three slice planes 72 at different positions along trajectory curve 62 throughout the outflow tract. Adapted geometrical model 32 is taken as a basis in Fig. 5, wherein slice plane determination unit 70 is also adapted to determine slice planes 72 based on enhanced adapted geometrical model 42.

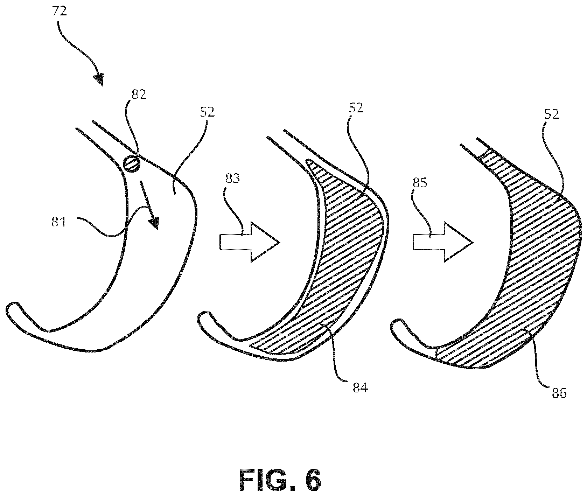

Fig. 6 exemplarily illustrates the operation of cross section determination unit 80. Each of the slice planes 72 extent over a range of the outflow tract and reach the edge of the outflow tract at some point. Cross section determination unit 80 determines the cross sectional area within the outflow tract for a particular slice plane 72. Fig. 6 illustrates one exemplary implementation of cross section determination unit 80, wherein different methods of determining an area within an arbitrary shape are well known to the skilled person and can readily be exchanged with the method exemplarily illustrated in Fig. 6. In Fig. 6 an area of the outflow tract in a particular slice plane 72 is determined by moving 81 a disc 82 within the area corresponding to outflow tract 52. After having passed the entire area, indicated as arrow 83, all points or pixels are labeled where disc 82 only touches the boundary of outflow tract 52. After that, indicated with arrow 85, dilate 86 is applied to compensate for shrinking.

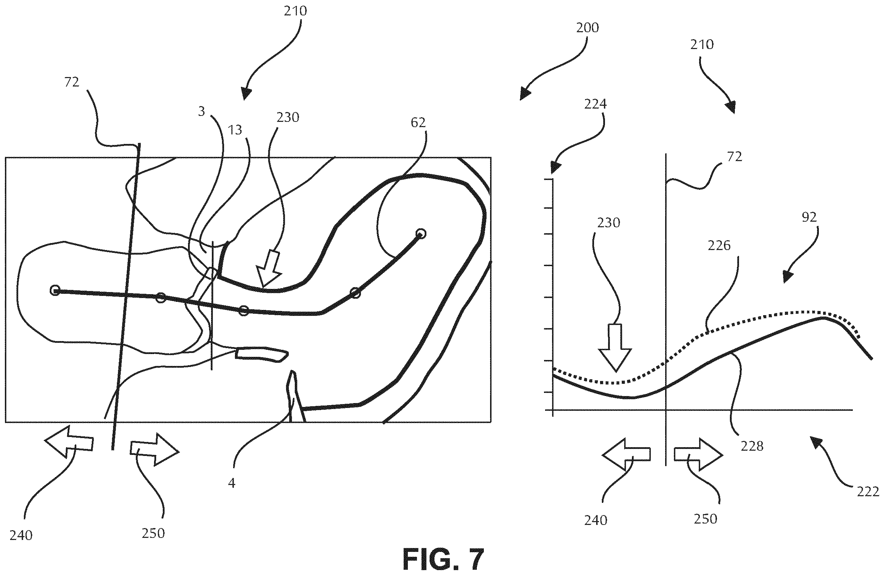

Fig. 7 schematically and exemplarily illustrates the operation of elevation profile determination unit 90 in an example of a user interface 200 presented by, for instance, a general purpose computer, a laptop, and so on. User interface 200 comprises a slice view 210 and an elevation profile view 220. Elevation profile determination unit 90 determines an elevation profile 92, which is displayed in elevation profile view 220. Elevation profile 92 indicates the course of a parameter relating to the slice plane 72 for different points of the trajectory curve 62. Elevation profile view 220 provides, along a horizontal axis 222, a position along trajectory curve 62 and, on a vertical axis 224, a value of the parameter underlying elevation profile 92. For instance, in Fig. 7 the parameter is the area obtained by cross section determination unit 80. As curve 226, the cross section of adapted geometrical model 32, i.e. e.g. before mitral valve implantation, is provided. With curve 228, the cross section of enhanced adapted geometrical model 42, i.e. e.g. after mitral valve implantation, is illustrated. It can be seen from elevation profile view 220, that the cross section of curve 228 is smaller, i.e. the outflow tract is obstructed, compared with curve 226. A minimum point in cross section along trajectory curve 62 is indicated with an arrow 230. Trajectory curve 62 and the arrow indicated as arrow 230 is also illustrated in slice view 210. Further, both slice view 210 and elevation profile view 220 illustrate a corresponding slice plane 72, which can be moved along trajectory curve 62. In the example of Fig. 7, slice plane 72 can, for instance, be moved with arrows 240 and/or 250.

However, user interface 200 is just an example of a user interface, and additions, modifications and so on can of course be considered by the skilled person.

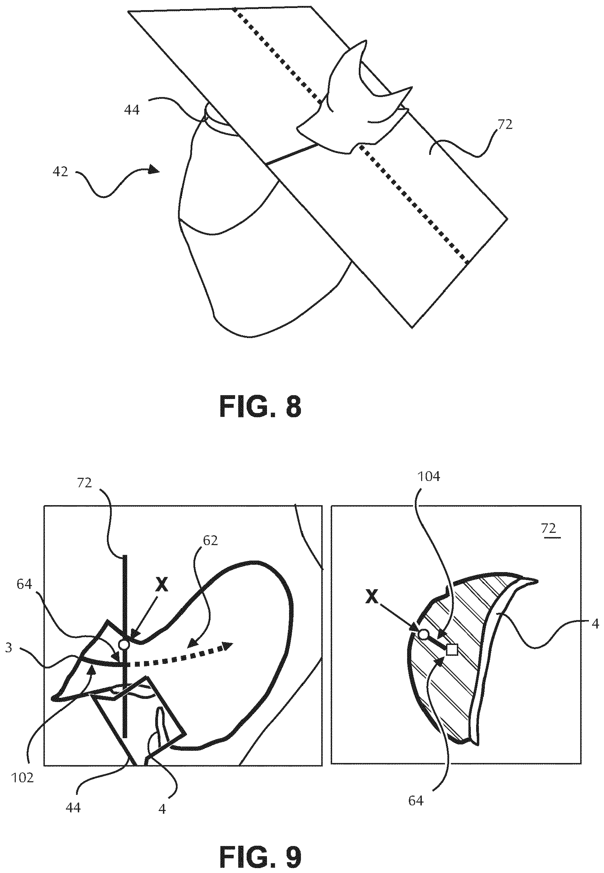

The function of weighting function determination unit 100 is explained with reference to Figs. 8 and 9. Fig. 8 schematically and exemplarily illustrates a perspective view on enhanced adapted geometric model 42, which is intersected by a slice plane 72. In order to compensate for rather non-linear effects of fluid mechanics, a weighting function w (p,X) is defined to be a non constant function with respect to a position X with a set of parameters p. The overall goal is to integrate over the weighting function for the volume within the outflow tract, based on both the adapted geometrical model 32 and the enhanced adapted geometrical model 42, so as to approximate the relative pressure drop between aorta and left ventricle before and after mitral valve replacement from a difference between both volume integrals. The definition of the weighting function is driven by the observation that obstructions affect the blood flow more if they are closer to the aortic valve and closer to the center line of the outflow tract. Accordingly, the definition of the weighting function may be pictured by a plane, i.e. slice plane 72, that is swapped along a center line, i.e. trajectory curve 62, of the outflow tract volume as illustrated in Fig. 9. As described earlier, trajectory curve 62 is a parametric curve that is constructed based on landmarks of the anatomical model of the heart. The curve starts, for instance, at the aortic valve 3 and ends at the apex 6.

Fig. 9 schematically and exemplarily illustrates the definition of contributions to a weighting function in two planes. Since it has been observed that obstructions can be described as a parameterized function depending on, among others, a longitudinal and a lateral distance, for each point X in the outflow tract 52, a longitudinal distance 102 can be defined as a distance along trajectory curve 62 between the center of aortic valve plane 14 and the intersection of slice plane 72 with trajectory curve 62, referred to as point 64 in Fig. 9. On the right side of Fig. 9 an in-plane view of slice plane 72 is shown, which illustrates a lateral distance 104 between point X and central point 64. The overall weighting function can then preferentially be described as a sum of two non linear functions, called part-weighting functions, wherein both functions depend on a set of parameters p and one of longitudinal distance 102 and lateral distance 104, respectively.

In other words, those two part-weighting functions depend on only one of the longitudinal and the lateral obstruction contribution. However, in other embodiments and depending on the parameters p obtained from calibration, only a single weighting function depending on the parameters p and both longitudinal distance 102 and lateral distance 104 can be determined.

In order to calibrate each of the part-weighting functions, in a simple case, polynomial functions can be assumed. Such function pattern covers a wide range of possible relationships between longitudinal and/or lateral distance and the effect on the pressure drop between aorta and left ventricle 5. One example would be a linear relation between the weight and the longitudinal and lateral distance. The parameters could be learnt by performing cross validation over a preselected set of functions and parameter rangers.

In a different embodiment, instead of calibrating and adjusting the weighting function, also the interval limits of integrating the weighting functions could be optimized or adjusted within a calibration step. In terms of the approach concerning trajectory curve 62, it could be contemplated to adjust the start and end points along trajectory curve 62 which define the integration interval, as can be seen, for instance in Fig. 4.

In one embodiment, obtaining the parameters for the weighting functions or the part-weighting functions, respectively, is based in the idea of calibrating the system based on flow simulations. A set of training examples including models with and without correctly placed implant models 44 is used as an input for a flow simulation. The training data set can preferentially be augmented by applying random deformations along the principal components of adapted heart models. The adapted heart models are, for instance, adapted geometrical model 32 and, in case implant model 44 is placed, also enhanced adapted geometrical model 42. Then, the simulation is set up such that the pressure drop between aortic valve 3 and left ventricle 5 is predicted. The result is assumed as ground truth and then used to determine the parameters of the weighting function based on, for instance, regression and cross validation.

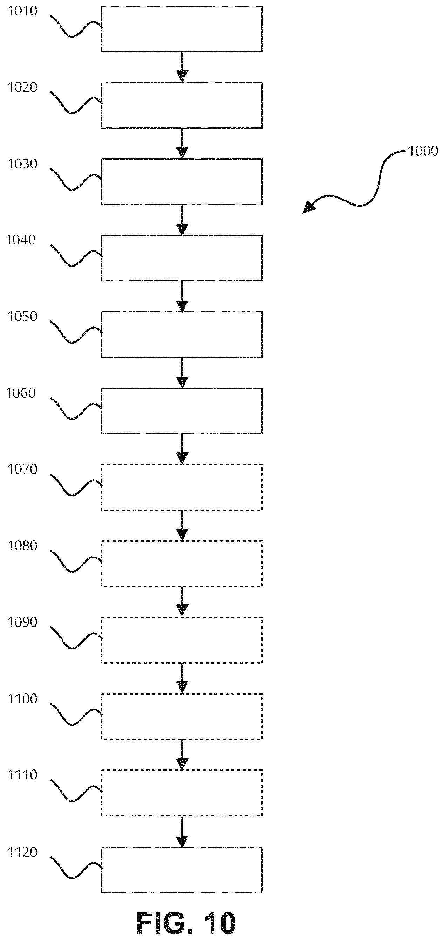

Fig. 10 schematically and exemplarily illustrates a flowchart according to a method 1000 for assessing outflow tract obstruction of a heart of a subject.

In step 1010, a geometrical model of the heart is provided. The geometrical model is, for instance, provided by geometrical model providing unit 10.

In step 1020, a volumetric image of the heart is provided. The volumetric image is, for instance, provided by volumetric image providing unit 20.

In step 1030, the geometrical model provided in step 1010 is adapted to obtain an adapted geometrical model 32, which is adapted to the volumetric image. The geometrical model is adapted, for instance, by first geometrical model adaptation unit 30.

In step 1040, an implant model 44 of an implant is provided. Implant model 44 is, for instance, provided by second geometrical model adaptation unit 40.

In step 1050, the implant model 44 is constructed into the adapted geometrical model 42 to obtain an enhanced adapted geometrical model 42. This step is preferentially also carried out by second geometrical model adaptation unit 40.

In step 1060, a trajectory curve 62 through an outflow tract 52 from at least one of the adapted geometrical model 32 and the enhanced adapted geometrical model 42 is determined.

In optional step 1070, a slice plane 72 corresponding to a point of the trajectory curve 62 determined in step 1060 is determined, wherein the normal of the slice plane coincides with a tangent to the trajectory curve 62 in this point.

In optional step 1080, a cross section of the outflow tract 52 based on the slice plane determined in step 1070 is determined.

In optional step 1090, an elevation profile 92 of a parameter relating to the slice plane 72 determined in step 1070 for different points of the trajectory curve 62 is determined. In one example, the parameter is the cross section determined in optional step 1080.

In optional step 1100, slice plane 72 and elevation profile 92 are simultaneously displayed to a user, wherein the point, to which slice plane 72 corresponds is indicated on elevation profile 92.

In optional step 1110, a weighting function is determined for at least one slice plane 72 based on at least one of the geometrical model, adapted geometrical model 32 and enhanced adapted geometrical model 42.

Finally, in step 1120, an outflow tract obstruction is assessed based on at least the adapted geometrical model 32, the enhanced adapted geometrical model 42 and the trajectory curve 62. The assessment is preferentially carried out by outflow tract obstruction assessing unit 50. In other examples, the assessment in step 1110 can further be based on at least one of the slice plane 72 determined in step 1070, the cross section determined in step 1080, the elevation profile determined in step 1090, the weighting function determined in step 1100 and an integral over the weighting function. This list is not exclusive and further parameters or the like, on which the assessment in step 1110 can be based, can be added by the skilled person.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

A single unit or device may fulfill the functions of several items recited in the claims. The mere fact that certain measures are recited in mutually different dependent claims does not indicate that a combination of these measures cannot be used to advantage.

Procedures like the provision of a geometrical model, the provision of a volumetric image, the adaptation of the geometrical model, the provision of an implant model, the construction of the implant model into the adapted geometrical model, the assessing of the outflow tract obstruction et cetera performed by one or several units or devices can be performed by any other number of units or devices. These procedures and/or the control of the system for assessing outflow tract obstruction of a heart of a subject in accordance with the method for assessing outflow tract obstruction of a heart of a subject can be implemented as program code means of a computer program and/or as dedicated hardware.

A computer program may be stored/distributed on a suitable medium, such as an optical storage medium or a solid-state medium, supplied together with or as part of other hardware, but may also be distributed in other forms, such as via the Internet or other wired or wireless telecommunication systems.

Any reference signs in the claims should not be construed as limiting the scope.

The present invention thus described relates to a system, a corresponding method and computer program for assessing outflow tract obstruction of a heart of a subject, wherein the system comprises a geometrical model providing unit 10 for providing a geometrical model of the heart; a volumetric image providing unit 20 for providing a volumetric image of the heart; a first geometrical model adaptation unit 30 for adapting the geometrical model to the volumetric image to obtain an adapted model 32; a second geometrical model adaptation unit 40 for providing an implant model 44 of an implant and for constructing the implant model 44 into the adapted model 32 to obtain an enhanced model 42; a trajectory curve determination unit 60 for determining a trajectory curve 62 through an outflow tract 52, and an outflow tract obstruction assessing unit 50 for assessing outflow tract obstruction based on the adapted model 32, the enhanced model 42 and the trajectory curve 62. The invention allows for an improved assessing of an outflow tract obstruction of a heart of a subject.

## Claims

1. A system for placing a valve implant and for assessing outflow tract obstruction of a heart of a subject, the system comprising:
- a geometrical model providing unit (10) for providing a geometrical model of the heart,
- a volumetric image providing unit (20) for providing a volumetric image of the heart,
- a first geometrical model adaptation unit (30) for adapting the geometrical model to the volumetric image to obtain an adapted geometrical model (32),
- a second geometrical model adaptation unit (40) for providing an implant model (44) of the implant and for constructing the implant model (44) into the adapted geometrical model (32) to obtain an enhanced adapted geometrical model (42),
- a trajectory curve determination unit (60) for determining a trajectory curve (62) through an outflow tract (52) from at least one of the adapted geometrical model (32) and the enhanced adapted geometrical model (42), and
- an outflow tract obstruction assessing unit (50) for assessing outflow tract obstruction based on the adapted geometrical model (32), the enhanced adapted geometrical model (42) and the trajectory curve (62), wherein
the trajectory curve (62) approximates a center line through the outflow tract (52) and comprises an interpolation, such as an interpolating spline curve, between anatomical landmarks, wherein
the anatomical landmarks include the center of the aortic valve plane (13), a center of the extruded mitral valve plane (14) and the apex (6), wherein the anatomical landmarks are comprised in the geometrical model and/or determined based on the geometrical model.

2. The system according to claim 1, wherein
the second geometrical model adaptation unit (40) is adapted to determine an annulus ring of a valve in the adapted geometrical model, wherein the annulus ring defines a plane comprising a center position and a normal vector, and to position the implant model with respect to the annulus ring.

3. The system according to claim 2, wherein
the second geometrical model adaptation unit (40) is adapted to allow a translation and/or rotation of the implant model (44) based on an input of a user.

4. The system according to claim 1, wherein
the second geometrical model adaptation unit (40) is adapted to provide the implant model (44) as a mesh template, wherein the mesh template is parameterized based on at least one of a size, width and length, and/or to construct a geometry based on an annulus ring geometry or a mitral valve (4) of the adapted geometrical model (32).

5. The system according to claim 1, wherein
the outflow tract obstruction assessing unit (50) is adapted to provide a relative quantity indicating the obstruction before and after mitral valve replacement based on the difference between the enhanced adapted geometrical model (42) and the adapted geometrical model (32) and based on the trajectory curve (62).

6. The system according to claim 1, wherein
the trajectory curve determination unit (60) is configured to determine the trajectory curve (62) based on the geometrical model of the heart from a central point of the aortic valve plane (13) to the apex (6).

7. The system according to claim 1, further comprising:
- a slice plane determination unit (70) for determining a slice plane (72) corresponding to a point of the trajectory curve (62), wherein the normal of the slice plane (72) coincides with a tangent to the trajectory curve (62) in the point, and
- a cross section determination unit (80) for determining a cross section of the outflow tract (52) based on the slice plane (72).

8. The system according to claim 7, further comprising:
- an elevation profile determination unit (90) for determining an elevation profile (92) of a parameter relating to the slice plane (72) for different points of the trajectory curve (62), and
- a display unit (200), wherein the display unit is configured to simultaneously display the slice plane (72) and the elevation profile (92) and to indicate the point, to which the slice plane (72) corresponds, on the elevation profile (92),
wherein the elevation profile (92) is preferentially based on the parameter for at least one of the adapted geometrical model (32) and the enhanced adapted geometrical model (42).

9. The system according to claim 8, wherein the parameter is at least one of an area, a perimeter or a compactness measure of the outflow tract in the slice plane (72), such as shape factors.

10. The system according to claim 7, further comprising:
- a weighting function determination unit (100) for determining a weighting function for at least one slice plane (72) based on at least one of the geometrical model, the adapted geometrical model (32) and the enhanced adapted geometrical model (42), wherein
the weighting function accounts for non-linear fluid mechanics of the outflow tract (52), and wherein
the outflow tract obstruction assessing unit (50) is adapted to assess the outflow tract obstruction based on the weighting function.

11. The system according to claim 10, wherein
the volume of the outflow tract is represented as a set of cross sections determined based on slice planes (72) along the trajectory curve (62), wherein
the outflow tract obstruction assessing unit (50) is adapted to determine an outflow tract geometry based on a volume integral of the weighting function over the outflow tract (52).

12. The system as defined in claim 10, wherein
the weighting function determination unit (100) is adapted to determine a parameterized weighting function and to calibrate the parameterized weighting function based on flow simulations such that as a calibration result the parameterized weighting function approximates the result of the flow simulation.

13. A computer-implemented method for placing a valve implant and for assessing outflow tract obstruction of a heart of a subject, the method comprising:
- providing (1010) a geometrical model of the heart,
- providing (1020) a volumetric image of the heart,
- adapting (1030) the geometrical model to the volumetric image to obtain an adapted geometrical model (32),
- providing (1040) an implant model (44) of the implant,
- constructing (1050) the implant model into the adapted geometrical model (32) to obtain an enhanced adapted geometrical model (42),
- determining (1060) a trajectory curve (62) through an outflow tract (52) from at least one of the adapted geometrical model (32) and the enhanced adapted geometrical model (42), and
- assessing (1120) outflow tract obstruction based on the adapted geometrical model (32), the enhanced adapted geometrical model (42) and the trajectory curve (62), wherein
the trajectory curve (62) approximates a center line through the outflow tract (52) and comprises an interpolation, such as an interpolating spline curve, between anatomical landmarks, wherein
the anatomical landmarks include the center of the aortic valve plane (13), a center of the extruded mitral valve plane (14) and the apex (6), wherein the anatomical landmarks are comprised in the geometrical model and/or determined based on the geometrical model.

14. A computer program for placing a valve implant and for assessing outflow tract obstruction of a heart of a subject, the computer program comprising program code means for causing a system as defined in claim 1 to carry out the method as defined in claim 13, when the computer program is run on the system.

## Patentansprüche

1. System zur Platzierung eines Herzklappenimplantats und zur Bewertung von Ausflusstraktobstruktion eines Herzens eines Patienten, wobei das System Folgendes umfasst:
- eine geometrische Modellbereitstellungseinheit (10) zur Bereitstellung eines geometrischen Modells des Herzens,
- eine volumetrische Bildbereitstellungseinheit (20) zur Bereitstellung eines volumetrischen Bilds des Herzens,
- eine erste geometrische Modellanpassungseinheit (30) zur Anpassung des geometrischen Modells an das volumetrische Bild, um ein angepasstes geometrisches Modell (32) zu erlangen,
- eine zweite geometrische Modellanpassungseinheit (40) zur Bereitstellung eines Implantatmodells (44) des Implantats und zum Hineinkonstruieren des Implantatmodells (44) in das angepasste geometrische Modell (32), um ein verbessertes angepasstes geometrisches Modell (42) zu erlangen,
- eine Trajektorienkurvenbestimmungseinheit (60) zum Bestimmen einer Trajektorienkurve (62) durch einen Ausflusstrakt (52) anhand von mindestens einem von dem angepassten geometrischen Modell (32) und dem verbesserten angepassten geometrischen Modell (42), und
- eine Ausflusstraktobstruktionsbewertungseinheit (50) zum Bewerten von Ausflusstraktobstruktion basierend auf dem angepassten geometrischen Modell (32), dem verbesserten angepassten geometrischen Modell (42) und der Trajektorienkurve (62), wobei
die Trajektorienkurve (62) eine Mittellinie durch den Ausflusstrakt (52) approximiert und eine Interpolation, zum Beispiel eine interpolierende Spline-Kurve, zwischen anatomischen Landmarken umfasst, wobei
die anatomischen Landmarken die Mitte der Aortenklappenebene (13), eine Mitte der extrudierten Mitralklappenebene (14) und die Herzspitze (6) umfassen, wobei die anatomischen Landmarken in dem geometrischen Modell enthalten sind und/oder basierend auf dem anatomischen Modell bestimmt werden.

2. System nach Anspruch 1, wobei
die zweite geometrische Modellanpassungseinheit (40) eingerichtet ist, um einen Annulusring einer Klappe in dem angepassten geometrischen Modell zu bestimmen, wobei der Annulusring eine Ebene definiert, die eine Mittenposition und einen Normalvektor umfasst, und um das Implantatmodell in Bezug auf den Annulusring zu positionieren.

3. System nach Anspruch 2, wobei
die zweite geometrische Modellanpassungseinheit (40) eingerichtet ist, um eine Translation und/oder Rotation des Implantatmodells (44) basierend auf einer Eingabe eines Benutzers zu erlauben.

4. System nach Anspruch 1, wobei
die zweite geometrische Modellanpassungseinheit (40) eingerichtet ist, um das Implantatmodell (44) als eine Netzvorlage bereitzustellen, wobei die Netzvorlage basierend auf mindestens einem von einer Größe, einer Breite und einer Länge parametrisiert ist, und/oder um eine Geometrie basierend auf einer Annulusringgeometrie oder einer Mitralklappe (4) des angepassten geometrischen Modells (32) zu konstruieren.

5. System nach Anspruch 1, wobei
die Ausflusstraktobstruktionsbewertungseinheit (50) eingerichtet ist, um eine relative Quantität bereitzustellen, die die Obstruktion vor und nach dem Mitralklappenersatz basierend auf dem Unterschied zwischen dem verbesserten angepassten geometrischen Modell (42) und dem angepassten geometrischen Modell (32) und basierend auf der Trajektorienkurve (62) angibt.

6. System nach Anspruch 1, wobei
die Trajektorienkurvenbestimmungseinheit (60) konfiguriert ist, um die Trajektorienkurve (62) basierend auf dem geometrischen Modell des Herzens von einem zentralen Punkt der Aortenklappenebene (13) aus zur Herzspitze (6) zu bestimmen.

7. System nach Anspruch 1, ferner umfassend:
- eine Schichtebenenbestimmungseinheit (70) zum Bestimmen einer Schichtebene (72), die einem Punkt der Trajektorienkurve (62) entspricht, wobei die Normale der Schichtebene (72) mit einer Tangente zur Trajektorienkurve (62) in dem Punkt zusammenfällt, und
- eine Querschnittbestimmungseinheit (80) zum Bestimmen eines Querschnitts des Ausflusstrakts (52) basierend auf der Schichtebene (72).

8. System nach Anspruch 7, ferner umfassend:
- eine Elevationsprofilbestimmungseinheit (90) zum Bestimmen eines Elevationsprofils (92) eines sich auf die Schichtebene (72) beziehenden Parameters für verschiedene Punkte der Trajektorienkurve (62), und
- eine Anzeigeeinheit (200), wobei die Anzeigeeinheit konfiguriert ist, um gleichzeitig die Schichtebene (72) und das Elevationsprofil (92) anzuzeigen und den Punkt, mit dem die Schichtebene (72) übereinstimmt, auf dem Elevationsprofil (92) anzugeben, wobei das Elevationsprofil (92) vorzugsweise auf dem Parameter für mindestens eines von dem angepassten geometrischen Modell (32) und dem verbesserten angepassten geometrischen Modell (42) basiert.

9. System nach Anspruch 8, wobei der Parameter mindestens eines von einer Fläche, einem Umfang oder einem Kompaktheitsmaß des Ausflusstrakts in der Schichtebene (72) ist, zum Beispiel Formfaktoren.

10. System nach Anspruch 7, ferner umfassend:
- eine Gewichtungsfunktionsbestimmungseinheit (100) zum Bestimmen einer Gewichtungsfunktion für mindestens eine Schichtebene (72) basierend auf mindestens einem von dem geometrischen Modell, dem angepassten geometrischen Modell (32) und dem verbesserten angepassten geometrischen Modell (42), wobei
die Gewichtungsfunktion nicht-lineare Strömungsmechanik des Ausflusstrakts (52) berücksichtigt, und wobei
die Ausflusstraktobstruktionsbewertungseinheit (50) dafür ausgelegt ist, die Ausflusstraktobstruktion basierend auf der Gewichtungsfunktion zu bewerten.

11. System nach Anspruch 10, wobei
das Volumen des Ausflusstrakts als ein Satz von Querschnitten dargestellt wird, die basierend auf Schichtebenen (72) entlang der Trajektorienkurve (62) bestimmt werden, wobei
die Ausflusstraktobstruktionsbewertungseinheit (50) dafür ausgelegt ist, eine Ausflusstraktgeometrie basierend auf einem Volumenintegral der Gewichtungsfunktion über den Ausflusstrakt (52) zu bestimmen.

12. System nach Anspruch 10, wobei
die Gewichtungsfunktionsbestimmungseinheit (100) dafür ausgelegt ist, eine parametrisierte Gewichtungsfunktion zu bestimmen und die parametrisierte Gewichtungsfunktion basierend auf Strömungssimulationen zu kalibrieren, sodass ein Kalibrierergebnis der parametrisierten Gewichtungsfunktion das Ergebnis der Strömungssimulation approximiert.

13. Computerimplementiertes Verfahren zur Platzierung eines Herzklappenimplantats und zur Bewertung von Ausflusstraktobstruktion eines Herzens eines Patienten, wobei das Verfahren Folgendes umfasst:
- Bereitstellen (1010) eines geometrischen Modells des Herzens,
- Bereitstellen (1020) eines volumetrischen Bilds des Herzens,
- Anpassen (1030) des geometrischen Modells an das volumetrische Bild, um ein angepasstes geometrisches Modell (32) zu erlangen,
- Bereitstellen (1040) eines Implantatmodells (44) des Implantats,
- Hineinkonstruieren (1050) des Implantatmodells in das angepasste geometrische Modell (32), um ein verbessertes angepasstes geometrisches Modell (42) zu erlangen,
- Bestimmen (1060) einer Trajektorienkurve (62) durch einen Ausflusstrakt (52) anhand von mindestens einem von dem angepassten geometrischen Modell (32) und dem verbesserten angepassten geometrischen Modell (42), und
- Bewerten (1120) von Ausflusstraktobstruktion basierend auf dem angepassten geometrischen Modell (32), dem verbesserten angepassten geometrischen Modell (42) und der Trajektorienkurve (62), wobei
die Trajektorienkurve (62) eine Mittellinie durch den Ausflusstrakt (52) approximiert und eine Interpolation, zum Beispiel eine interpolierende Spline-Kurve, zwischen anatomischen Landmarken umfasst, wobei
die anatomischen Landmarken die Mitte der Aortenklappenebene (13), eine Mitte der extrudierten Mitralklappenebene (14) und die Herzspitze (6) umfassen, wobei die anatomischen Landmarken in dem geometrischen Modell enthalten sind und/oder basierend auf dem anatomischen Modell bestimmt werden.

14. Computerprogramm zur Platzierung eines Herzklappenimplantats und zur Bewertung von Ausflusstraktobstruktion eines Herzens eines Patienten, wobei das Computerprogramm Programmcodemittel zum Veranlassen eines Systems nach Anspruch 1 zum Durchführen des Verfahrens nach Anspruch 13 umfasst, wenn das Computerprogramm auf dem System ausgeführt wird.

## Revendications

1. Système pour mettre en place un implant de valvule et pour évaluer un obstacle à une voie de sortie du coeur d'un sujet, le système comprenant :
- une unité de fourniture de modèle géométrique (10) pour fournir un modèle géométrique du cœur,
- une unité de fourniture d'image volumétrique (20) pour fournir une image volumétrique du cœur,
- une première unité d'adaptation de modèle géométrique (30) pour adapter le modèle géométrique à l'image volumétrique afin d'obtenir un modèle géométrique adapté (32),
- une seconde unité d'adaptation de modèle géométrique (40) pour fournir un modèle géométrique (44) de l'implant et construire le modèle d'implant (44) dans le modèle géométrique adapté (32) afin d'obtenir un modèle géométrique adapté amélioré (42),
- une unité de détermination de courbe de trajectoire (60) pour déterminer une courbe de trajectoire (62) à travers une voie de sortie (52) d'au moins l'un du modèle géométrique adapté (32) et du modèle géométrique adapté amélioré (42), et
- une unité d'évaluation d'obstacle à la voie de sortie (50) pour évaluer un obstacle à la voie de sortie sur la base du modèle géométrique adapté (32), du modèle géométrique adapté amélioré (42) et de la courbe de trajectoire (62), dans lequel :
la courbe de trajectoire (62) se rapproche d'une ligne centrale à travers la voie de sortie (52) et comprend une interpolation telle qu'une courbe spline d'interpolation entre des repères anatomiques, dans lequel :
les repères anatomiques incluent le centre du plan des valvules aortiques (13), un centre du plan de la valvule mitrale refoulé (14) et la pointe (6), dans lequel les repères anatomiques sont compris dans le modèle géométrique et/ou déterminés sur la base du modèle géométrique.

2. Système selon la revendication 1, dans lequel :
la seconde unité d'adaptation de modèle géométrique (40) est à même de déterminer une couronne annulaire d'une valvule dans le modèle géométrique adapté, dans lequel la couronne annulaire définit un plan comprenant une position centrale et un vecteur normal et de positionner le modèle d'implant par rapport à la couronne annulaire.

3. Système selon la revendication 2, dans lequel :
la seconde unité d'adaptation de modèle géométrique (40) est à même de permettre une translation et/ou une rotation du modèle d'implant (44) sur la base d'une entrée d'un utilisateur.

4. Système selon la revendication 1, dans lequel :
la seconde unité d'adaptation de modèle géométrique (40) est à même de fournir le modèle d'implant (44) sous la forme d'un modèle maillé, dans lequel le modèle maillé est paramétré sur la base d'au moins l'une d'une taille, d'une largeur et d'une longueur et/ou de construire une géométrie basée sur une géométrie de couronne annulaire ou d'une valvule mitrale (4) du modèle géométrique adapté (32).

5. Système selon la revendication 1, dans lequel :
l'unité d'évaluation d'obstacle à la voie de sortie (50) est à même de fournir une quantité relative indiquant l'obstacle avant et après le remplacement d'une valvule mitrale sur la base de la différence entre le modèle géométrique adapté amélioré (42) et le modèle géométrique adapté (32) et sur la base de la courbe de trajectoire (62).

6. Système selon la revendication 1, dans lequel :
l'unité de détermination de courbe de trajectoire (60) est configurée pour déterminée la courbe de trajectoire (62) sur la base du modèle géométrique du coeur d'un point central du plan des valvules aortiques (13) à la pointe (6).

7. Système selon la revendication 1, comprenant en outre :
- une unité de détermination de plan de jonction (70) pour déterminer un plan de coupe (72) correspondant à un point de la courbe de trajectoire (62), dans lequel la normale du plan de coupe (72) coïncide avec une tangente à la courbe de trajectoire (62) au point, et
- une unité de détermination de section transversale (80) pour déterminer une section transversale de la voie de sortie (52) sur la base du plan de coupe (72).

8. Système selon la revendication 7, comprenant en outre :
- une unité de détermination de profil d'élévation (90) pour déterminer un profil d'élévation (92) d'un paramètre se rapportant au plan de coupe (72) pour différents points de la courbe de trajectoire (62), et
- une unité d'affichage (200), dans lequel l'unité d'affichage est configurée pour afficher simultanément le plan de coupe (72) et le profil d'élévation (92) et indiquer le point auquel le plan de coupe (72) correspond sur le profil d'élévation (92),
dans lequel le profil d'élévation (92) est de préférence basé sur le paramètre pour au moins l'un du modèle géométrique adapté (32) et du modèle géométrique adapté amélioré (42).

9. Système selon la revendication 8, dans lequel le paramètre est au moins l'un(e) d'une surface, d'un périmètre ou d'une mesure de compacité de la voie de sortie dans le plan de coupe (72), notamment des facteurs de forme.

10. Système selon la revendication 7, comprenant en outre :
- une unité de détermination de fonction de pondération (100) pour déterminer une fonction de pondération pour au moins un plan de coupe (72) sur la base d'au moins l'un du modèle géométrique, du modèle géométrique adapté (32) et du modèle géométrique adapté amélioré (42), dans lequel :
la fonction de pondération tient compte de la mécanique des fluides non linéaires de la voie de sortie (52), et dans lequel :
l'unité d'évaluation de l'obstacle à la voie de sortie (50) est à même d'évaluer l'obstacle à la voie de sortie sur la base de la fonction de pondération.

11. Système selon la revendication 10, dans lequel :
le volume de la voie de sortie est représenté sous la forme d'un ensemble de sections transversales déterminées sur la base de plans de découpe (72) le long de la courbe de trajectoire (62), dans lequel :
l'unité d'évaluation de l'obstacle à la voie de sortie (50) est à même de déterminer une géométrie de la voie de sortie sur la base d'un volume intégral de la fonction de pondération sur la voie de sortie (52).

12. Système selon la revendication 10, dans lequel :
l'unité de détermination de la fonction de pondération (100) est à même de déterminer une fonction de pondération paramétrée et de calibrer la fonction de pondération paramétrée sur la base de simulations d'écoulement de sorte que, en tant que résultat de calibrage, la fonction de pondération paramétrée se rapproche du résultat de la simulation d'écoulement.

13. Procédé mis en oeuvre par ordinateur pour placer un implant valvulaire et évaluer l'obstacle à une voie de sortie d'un coeur d'un sujet, le procédé comprenant :
- la fourniture (1010) d'un modèle géométrique du coeur,
- la fourniture (1020) d'une image volumétrique du coeur,
- l'adaptation (1030) du modèle géométrique à l'image volumétrique pour obtenir un modèle géométrique adapté (32),
- la fourniture (1040) d'un modèle (44) de l'implant,
- la construction (1050) du modèle d'implant dans le modèle géométrique adapté (32) pour obtenir un modèle géométrique adapté amélioré (42),
- la détermination (1060) d'une courbe de trajectoire (62) à travers une voie de sortie (52) d'au moins l'un du modèle géométrique adapté (32) et du modèle géométrique adapté amélioré (42), et
- l'évaluation (1120) d'un obstacle à la voie de sortie sur la base du modèle géométrique adapté (32), du modèle géométrique adapté amélioré (42) et de la courbe de trajectoire (62), dans lequel :
la courbe de trajectoire (62) se rapproche d'une ligne centrale à travers la voie de sortie (52) et comprend une interpolation, telle qu'une courbe spline d'interpolation, entre des repères anatomiques, dans lequel :
les repères anatomiques incluent le centre du plan des valvules aortiques (13), un centre du plan de valvule mitral refoulé (14) et la pointe (6), dans lequel les repères anatomiques sont compris dans le modèle géométrique et/ou déterminés sur la base du modèle géométrique.

14. Programme d'ordinateur pour placer un implant valvulaire et pour évaluer un obstacle à la voie de sortie d'un coeur d'un sujet, le programme d'ordinateur comprenant des moyens de codage de programme pour amener un système selon la revendication 1 à effectuer le procédé selon la revendication 13 lorsque le programme d'ordinateur court sur le système.
